# EUROPEAN PATENT APPLICATION

(11) **EP 4 574 202 A1**
(43) Date of publication of application: **25.06.2025**
(21) Application number: 23855114.7
(22) Date of filing: 11.08.2023
(51) Int. Cl.: A61N 2/02, A61N 2/00, A61B 5/055, A61B 5/00

(54) **MAGNETIC RESONANCE IMAGING-BASED ALTERNATING MAGNETIC FIELD TREATMENT SYSTEM**

(30) Priority: 17.08.2022 KR 20220102819
(71) Applicant: Seoul National University R&DB Foundation, Seoul 08826 (KR); Brain & Beyonds Co., Ltd., Seoul 03122 (KR); Korea University Research and Business Foundation, Seoul 02841 (KR); Gachon University of Industry-Academic Cooperation Foundation, Seongnam-si, Gyeonggi-do 13120 (KR)
(72) Inventor: PAEK, Sun Ha, Seoul 05274 (KR); CHO, Zang Hee, Hwaseong-si Gyeonggi-do 18322 (KR); SON, Young Don, Incheon 21982 (KR); KWON, Dae Hyuk, Seoul 05242 (KR); PARK, Soon Beom, Seoul 05274 (KR)
(74) Representative: Algemeen Octrooi- en Merkenbureau B.V.
(86) International application number: PCT/KR2023/011964
(87) International publication number: WO 2024/039147

(57) **Abstract**

A magnetic resonance imaging (MRI)-based alternating magnetic field (AMF) treatment system according to one embodiment of the technical idea of the present invention comprises an MRI device including an MRI coil system, and is configured to generate an AMF by applying an alternating current to the MRI coil system in the MRI device.

## Description

### TECHNICAL FIELD

The present disclosure relates to a device and method capable of performing alternating magnetic field (AMF) treatment on a subject for examination and treatment with the aid of a magnetic resonance imaging (MRI) system, and more particularly, to an MRI-based AMF treatment system and method that uses coils in an MRI system so as to apply an AMF of a particular frequency to a target area of a body in order to treat diseases such as tumors in the body.

### BACKGROUND

Despite the advancements in modern medicine, cancer remains one of the most difficult diseases to treat among human diseases. Cancer is a tissue formed by aggregation of abnormal cells and develops in one or more tissues and spreads to other tissues. As of now, there are more than 100 known types of cancer.

It is known that 90 to 95% of the main causes of cancer are genetic mutations caused by environmental factors, and most of these factors include smoking, obesity, radiation, UV, stress, environmental pollution, etc.

Since cancer has a rapid growth rate and a strong ability to invade surrounding tissues, and thus, the boundary with normal tissues is not clear, it is necessary to remove the tumor and surrounding tissues as much as possible through surgical operation while minimizing damage to the body's functions. It is not easy to completely remove a tumor through surgical operation, and there are adjuvant treatment methods such as radiation therapy, metabolic antagonists, chemotherapy, targeted therapy, immunotherapy, or parallel treatments of these.

However, these conventional methods generally have side effects such as hair loss, bone marrow suppression, drug resistance, and organ damage, although they may vary from patient to patient. Numerous efforts are being made around the world to overcome these side effects and conquer cancer, but the average survival period of cancer is still short and the possibility of recurrence is high, making the development of new treatments urgent.

Many basic and clinical medical studies are being conducted continuously and various treatment methods have been presented but it is still difficult to cure cancer, and most basic medical studies are being conducted continuously towards inhibiting in biological or chemical methods to inhibit the rapid growth and invasion of cancer.

In this regard, if magnetic nanoparticles are administered to a target site where a tumor is located and an AMF is applied to the site, heat is generated from the magnetic nanoparticles that move along the AMF, and a treatment method that reduces the size of a tumor through this heat-generating effect has recently been devised. However, if magnetic nanoparticles such as iron oxide particles were administered into the human body, they could not be recovered, and thus, there was a possibility that other fatal side effects could be resulted in, and there was a drawback of not being able to avoid a phenomenon in which the site in question was colored darkly in appearance due to the characteristics of the magnetic nanoparticles. As there is a continued demand for technology that achieves AMF treatment that exhibits therapeutic effects without the use of magnetic nanoparticles so as to solve the problems of AMF treatment using such existing nanoparticles and to minimize side effects on the human body, the coil system of an MRI device can provide conditions that allow for the formation of an AMF around a treatment target.

Since conventional magnetic stimulation devices generally use one or more coils arranged in parallel, three-dimensional magnetic stimulation and magnetic stimulation that fits the stimulation volume are limited by placing the device at the location to be stimulated and using only one side. The magnetic field of an MRI system is formed using a superconductor coil that wraps around a cylindrical bore and forms a strong magnetic field inside the bore, a gradient coil that applies gradients to the magnetic field strength in the x-, y-, and z-axis directions so that the spins of the nuclei in the examination subject can be encoded according to their position, and a high-frequency multi-coil that can transmit and receive the resonance and reception signals of radio frequency (RF) generated from the nuclear spins of the examination subject. Therefore, the magnetic fields used in the MRI system include a uniform main magnetic field formed in the bore that allows the whole body of a patient to be positioned inside thereof, a gradient magnetic field generated by a pair of gradient coils positioned around the whole body of the patient, and a local RF magnetic field generated from RF coils.

### SUMMARY

### TECHINCL OBJECTS

The present disclosure is intended to provide an MRI-based AMF treatment system.

Specifically, the present disclosure is intended to provide an MRI-based AMF treatment system that forms a fusion-type system by sharing an MRI coil system to convert a surrounding magnetic field into an AMF by flowing an alternating current through an MRI coil.

In addition, the present disclosure is intended to provide an MRI-based AMF treatment system in which an MRI gradient coil as a magnetic field generating device is formed to have a sufficiently large inner diameter so as to completely surround the whole body of a patient.

Further, the present disclosure is intended to provide an MRI-based AMF treatment system that can prevent the sensitivity of AMF treatment from being reduced by enabling proximity stimulation by positioning RF coils of an MRI system that can surround various local sites of a body.

Moreover, the present disclosure is intended to provide an MRI-based AMF treatment system that can perform AMF treatment and MRI scanning with a time difference in a shuttle fashion by manufacturing an MRI device and an AMF device independently.

In addition, the present disclosure is intended to provide an MRI-based AMF treatment system that can achieve two objectives of diagnosis and treatment of the body of a patient by performing MRI scanning as soon as AMF treatment is completed, and can help set goals for AMF treatment and monitor the progress of treatment.

Further, the technical objects to be achieved by the MRI-based AMF treatment system according to one embodiment of the technical idea of the present disclosure are not limited to the objects for solving the problems mentioned above, and other objects that have not been mentioned will be clearly understood by those having ordinary skill in the art from the description below.

### TECHNICAL SOLUTION

A magnetic resonance imaging (MRI)-based alternating magnetic field (AMF) treatment system according to one embodiment of the technical idea of the present disclosure includes an MRI device including an MRI coil system, wherein the MRI-based AMF treatment system is configured to generate an AMF by applying an alternating current to the MRI coil system in the MRI device.

A magnetic resonance imaging (MRI)-based alternating magnetic field (AMF) treatment system according to another embodiment of the technical idea of the present disclosure includes an MRI device; and an AMF generating part operable in conjunction with the MRI device and configured to generate an AMF, wherein the system is configured to operate between an MRI scanning mode and an AMF treatment mode. Here, the system may be an integrated system (a system in which the MRI scanning part and the AMF generating part are integrated) configured so that MRI scanning and AMF treatment are performed in a single space, or a fusion-type system (a system in which the MRI scanning part and the AMF generating part are separated and operable) in which the MRI scanning by the MRI device and the AMF treatment by the AMF generating part are performed independently of each other in each of the separate spaces, but these spaces are combined to enable reciprocating movement between them.

The MRI device in the integrated system includes: an MRI coil system configured to surround a target area of a whole body of a patient and generate an AMF; a signal transmission/reception part configured to apply an alternating current to the MRI coil system; and a system control part configured to switch between the MRI scanning mode and the AMF treatment mode and to adjust a strength and a frequency of the AMF.

The AMF generating part in the fusion-type system includes an AMF coil system configured to surround a target area of a whole body of a patient and generate an AMF, and the MRI-based AMF treatment system further includes: a shuttle bed configured to be movable to separated spaces according to the MRI scanning mode and the AMF treatment mode; a signal transmission/reception part configured to apply an alternating current to at least the AMF coil system; and a system control part configured to control operation of the shuttle bed, the MRI device, and the AMF generating part and to adjust a strength and a frequency of the AMF according to each mode switching. Here, the AMF coil system may include at least one or more of a gradient coil and an RF coil, as with the coil system of the MRI device.

The MRI coil system includes at least one or more of a gradient coil and an RF coil.

The gradient coil is configured to surround the target area of the whole body of the patient and change magnetic fields in x, y, and z axes three-dimensionally, and the RF coil is configured to surround a local target area of the patient and generate an AMF.

The coil may be arranged in a plurality of parallel structures.

The coil may be configured to have a plurality of different inner diameters.

The MRI-based AMF treatment system is configured to operate in one of the MRI scanning mode and the AMF treatment mode, and to operate the signal transmission/reception part via an existing MRI sequence controller when operating in the MRI scanning mode, and to transmit a signal to and operate the gradient coil or the RF coil according to a sequence designed separately so that an alternating current is supplied to an MRI coil when operating in the AMF treatment mode.

The system control part is configured to flow an alternating current having a set frequency through the gradient coil or the RF coil, convert a surrounding magnetic field into an AMF, and thereby generate the AMF in the target area of the whole body of the patient.

The set frequency has an electromagnetic wave frequency range of 380 kHz or less that does not have a harmful effect on a human body.

The system control part controls one of the MRI scanning mode and the AMF treatment mode to be closed in a switched-on or switched-off control method.

The system control part controls a table on which the patient is positioned so that an MRI scanning site is drawn into an isocenter of a static magnetic field when the MRI-based AMF treatment system completes driving the AMF treatment mode, and controls the table on which the patient is positioned to be drawn out of the MRI-based AMF treatment system when treatment and diagnosis via the MRI-based AMF treatment system are completed.

A method of operating an MRI-based AMF treatment system according to one embodiment of the technical idea of the present disclosure includes selecting to operate in one of an MRI scanning mode and an AMF treatment mode according to an input user command; and controlling a position of a table according to a selected mode.

A signal transmission/reception part is operated via an existing MRI sequence controller when operating in the MRI scanning mode, and a signal is transmitted to a coil according to a sequence designed to supply an alternating current to the coil configured to generate an AMF, thereby operating the coil when operating in the AMF treatment mode.

The method, in the case of selecting the AMF treatment mode, includes controlling the position of the table so that a target area of a body is located at a center of a coil for generating an AMF; setting parameters including a strength and a frequency of an AMF waveform; and generating an electromagnetic wave signal having a set AMF waveform through the coil.

The method further includes, after the generating the electromagnetic wave signal having the set AMF waveform, controlling the position of the table so that the target area of the body is located at a center of a static magnetic field of an MRI device; and performing an MRI scanning operation.

MRI scanning and AMF treatment in the method may be configured to be performed in a single space.

The table is a table of an MRI device, and the coil is an MRI coil of the MRI device.

The MRI scanning and AMF treatment in the method may be configured to be performed in each of spaces that are spaced and separated from each other, and reciprocating movement between these spaces is possible.

A position control of the table comprises being performed by a shuttle bed configured to be movable through separated spaces according to the MRI scanning mode and the AMF treatment mode.

As such, the MRI-based AMF treatment system according to one embodiment of the technical idea of the present disclosure is configured to enable separate and fusion operations according to clinical needs by configuring the AMF treatment device for treatment only and the existing diagnostic MRI independently. The MRI coil system (e.g., the gradient coil and/or the RF coil) installed in a typical MRI device can be applied to the AMF treatment device of the system to thereby generate an alternating magnetic field but is not necessarily limited thereto, and any coil system that generates an alternating magnetic field can be applied.

The method of operating such a fusion-type MRI-based AMF treatment system further includes, after outputting an electromagnetic wave signal having the set AMF waveform or performing MRI scanning, controlling the position of a shuttle bed capable of moving reciprocatingly between the two devices for the connected MRI scanning or AMF treatment; and coupling a patient transfer support on the shuttle bed with a patient table.

A non-transitory computer-readable medium according to one embodiment of the technical idea of the present disclosure includes instructions that cause a processor to perform the method of operating described above.

### EFFECTS OF THE DISCLOSURE

By providing the MRI-based AMF treatment system according to one embodiment of the technical idea of the present disclosure, there is an effect of being able to reduce side effects in the treatment of diseases such as tumors by applying only an AMF to a target area of the body without using separate magnetic nanoparticles.

In addition, by providing the MRI-based AMF treatment system according to one embodiment of the technical idea of the present disclosure, there is an advantage of being able to form an integrated system by sharing the MRI coil system to convert a surrounding magnetic field into an AMF by flowing an alternating current through the MRI coil.

Further, by providing the MRI-based AMF treatment system according to one embodiment of the technical idea of the present disclosure, there is also an advantage of being able to use the MRI gradient coil having a sufficiently large inner diameter so as to completely surround the whole body of a patient as the magnetic field generating device.

Moreover, by providing the MRI-based AMF treatment system according to one embodiment of the technical idea of the present disclosure, there is also an advantage of being able to prevent the sensitivity of AMF treatment from being reduced by enabling proximity stimulation by positioning the RF coil of the MRI system that can surround various local sites of the body.

Furthermore, the present disclosure also has advantages of being able to achieve two objectives of treatment and diagnosis of the body of a patient by performing MRI scanning as soon as AMF treatment is completed, and to help set goals for AMF treatment and monitor the progress of treatment.

In addition, by providing the MRI-based AMF treatment system according to one embodiment of the technical idea of the present disclosure, the AMF treatment device for treatment only and the existing MRI device for diagnosis can be configured independently, and the fusion-type system capable of separate and fusion operations according to clinical needs can be configured via the shuttle bed that can move reciprocatingly between the two devices, which leads to an advantage of high efficiency and maximum profit operation.

Further, the effects that can be achieved by providing the MRI-based AMF treatment system according to one embodiment of the technical idea of the present disclosure are not limited to those mentioned above, and other effects that have not been mentioned can be clearly appreciated by those having ordinary skill in the art from the description below.

### BRIEF DESCRIPTION OF THE DRAWINGS

For a more complete understanding of the drawings cited herein, a brief description of each drawing is provided.
FIG. 1 is a schematic diagram of an integrated system according to one embodiment of the present disclosure including a typical MRI device;
FIG. 2 is a diagram shown to describe a method of switching between an MRI scanning mode and an AMF treatment mode using an MRI RF system according to one embodiment of the present disclosure;
FIG. 3 is one example of an alternating voltage waveform generated by RF coils that can be used in an MRI-based AMF treatment system according to one embodiment of the present disclosure;
FIG. 4 shows a sinusoidal gradient waveform that can be used in the MRI-based AMF treatment system according to one embodiment of the present disclosure;
FIG. 5 is a diagram showing by way of example the types of gradient coils in a form fixed to an MRI gantry and a whole-body RF coil that can be used in the MRI-based AMF treatment system according to one embodiment of the present disclosure;
FIG. 6 is a diagram showing some example forms of detachable local RF coils that can be used in the MRI-based AMF treatment system according to one embodiment of the present disclosure;
FIG. 7a is a diagram showing a solenoid coil structure of volume coils of the detachable local RF coils that can be used in the MRI-based AMF treatment system according to one embodiment of the present disclosure;
FIG. 7b is a diagram showing a saddle-type Helmholtz coil structure of the volume coils of the detachable local RF coils that can be used in the MRI-based AMF treatment system according to one embodiment of the present disclosure;
FIG. 7c is a diagram showing a birdcage coil structure of the volume coils of the detachable local RF coils that can be used in the MRI-based AMF treatment system according to one embodiment of the present disclosure;
FIG. 7d is a diagram showing the configuration of a possible surface coil of the detachable local RF coils that can be used in the MRI-based AMF treatment system according to one embodiment of the present disclosure;
FIG. 7e is a diagram showing an 8-channel phased array coil in the form in which multiple surface coils are arranged that can be used in the MRI-based AMF treatment system according to one embodiment of the present disclosure;
FIG. 8 is a diagram shown to describe a fusion operation through a reciprocating movement of a shuttle bed of a fusion-type system according to one embodiment of the present disclosure;
FIG. 9 shows a flowchart of a treatment and diagnosis process in an integrated MRI-based AMF treatment system according to one embodiment of the present disclosure; and
FIG. 10 shows a flowchart of a treatment and diagnosis process in a fusion-type MRI-based AMF treatment system according to one embodiment of the present disclosure.

### DETAILED DESCRIPTION OF EMBODIMENTS

As the technology disclosed herein can be subject to various modifications and have several embodiments, particular embodiments are shown in the drawings and will be described in detail through the detailed description. However, this is not intended to limit the technology disclosed herein to particular embodiments, and it should be understood that the technology disclosed herein includes all modifications, equivalents, and alternatives that fall within the spirit and scope of the technology disclosed herein.

In describing the technology disclosed herein, if it is determined that a specific description of a related known technology may unnecessarily obscure the gist of the technology disclosed herein, the detailed description thereof will be omitted. In addition, the numbers (e.g., first, second, etc.) used in the course of describing the present specification are merely identifiers for distinguishing one component from another.

Further, if a component is referred to as being "connected," "coupled," or the like with another component herein, it should be understood that the one component may be connected or coupled directly with said another component, but they may also be connected or coupled via yet another component in-between unless there is a description to the contrary.

Moreover, for a component represented by '~ part' herein, two or more components may be combined into one component, or one component may be divided into two or more components according to further subdivided functions. In addition, each of the components to be described below may additionally perform some or all of the functions for which another component is responsible in addition to the main functions for which it is responsible, and some of the main functions for which each of the components is responsible may be performed exclusively by another component, as a matter of course.

Expressions such as "first" or "second" used in various embodiments may modify diverse components, regardless of order and/or importance, and do not limit the components. For example, a first component may be named a second component, and similarly, a second component may also be renamed as a first component, without departing from the scope of the technology disclosed herein.

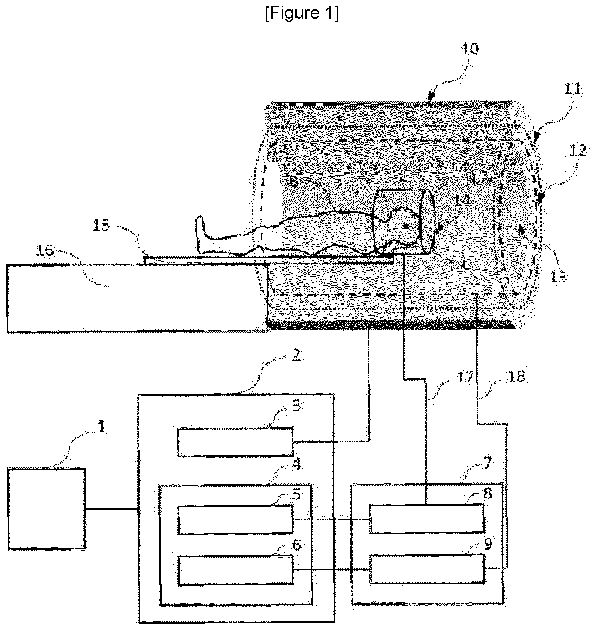

A method of treating a disease such as a tumor by applying an AMF to a body requires a need to further enhance the safety and effectiveness of the treatment method by applying a constant AMF to a target area of the body. One embodiment of the present disclosure provides an integrated MRI-based AMF treatment system that can more effectively apply an AMF to a target area of the body and efficiently reduce the size of a tumor or inhibit the growth of a tumor in a shorter period of time by utilizing coils that are already present in a common MRI system (e.g., see FIG. 1). Further, a device and method for treating cancer using an AMF is described in detail in Korean Patent Application No. 10-2022-0059039 (Alternating Magnetic Field Generator for Cancer Treatment) filed by the present applicant, the entire specification of which is incorporated herein by reference.

Referring to FIG. 1, the MRI-based AMF treatment system may largely include an operating computer 1, a system control part 2, a signal transmission/reception part 7, and an MRI gantry 10, and the whole body of a patient may be positioned on a table that can be inserted inside along the horizontal axis of a cylindrical bore 13.

The operating computer 1 may instruct the system control part 2 to control the operation of the entire MRI-based AMF treatment system, and at the same time, select between an MRI scanning mode and an AMF treatment mode, and parameters and signal sequence information required for each mode. Further, such an operating computer may be configured integrally with the system control part described later.

The system control part 2 may include a gantry control part 3 that controls various electronic devices of the MRI gantry 10, and a sequence control part 4 that controls the sequence of signals formed in the gantry 10.

The sequence control part 4 may include an RF coil control part 5 that controls an RF coil signal transmission/reception part 8 and a gradient coil control part 6 that can control a gradient coil signal transmission part 9. The sequence control part 4 may control the RF coil signal transmission/reception part 8 and the gradient coil signal transmission part 9 according to parameters such as the strength, start time, end time, etc., of the signal received from the operating computer 1, and a series of control sequences.

The signal transmission/reception part 7 may include the RF coil signal transmission/reception part 8, and the gradient coil signal transmission part 9 including an amplifier for a gradient coil transmission signal, and can change the magnetic field formed by an MRI coil system including gradient coils 51, 52, and 53 and RF coils 14, 54, and 61 according to the signal parameters and control sequences described above.

The gradient coils 51, 52, and 53 in the MRI gantry 10 may be generally located inside 12 of a main magnetic field coil level 11 that forms a static magnetic field in the bore 13, and the RF coils 14, 54, and 61 may be located close to the body, except for a whole-body RF coil 54 embedded in the gantry 10 as with the gradient coils 51, 52, and 53, and can change the static magnetic field in the bore 13.

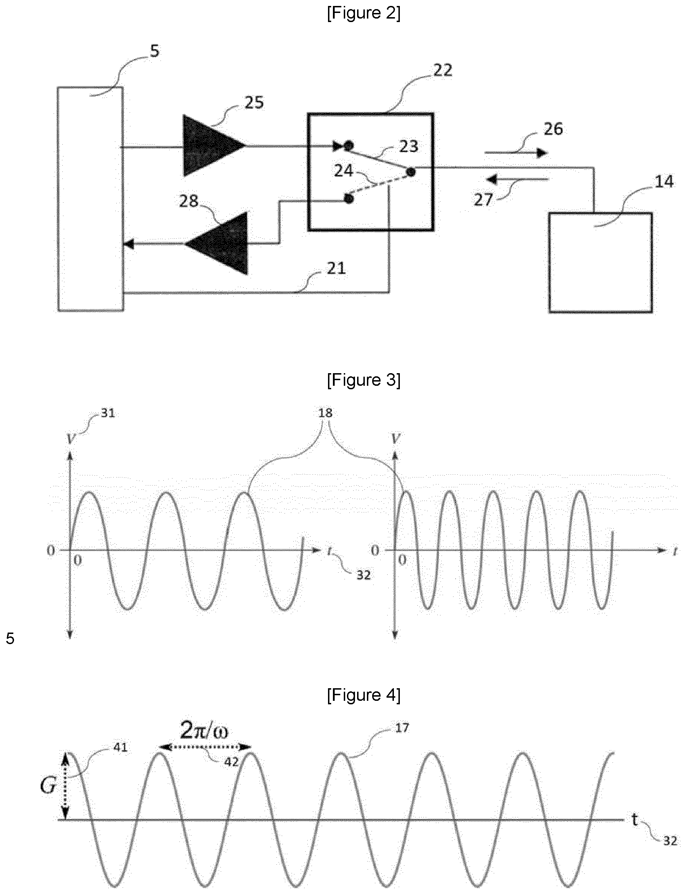

FIG. 2 is a diagram showing in detail the RF coil signal transmission/reception part 8 included in the MRI-based AMF treatment system of FIG. 1.

Referring to FIG. 2, if, for example, an RF coil for both transmission and reception is utilized in MRI image scanning, an RF transmission signal produced by the RF coil control part 5 is amplified into an RF coil transmission signal 26 via an amplifier 25 and then applied to the human body via the RF coils 14 and 61. The hydrogen atoms in the human body that resonate with the RF signal generate an MR signal 27, which is received by the RF coils and input into the computer via an RF reception signal amplifier 28. At this time, a transmission/reception switch 22 can adjust the transmission/reception direction of the RF signal and the MR signal. The RF coil control part 5 adjusts so as to be in connected 23 and disconnected 24 states when transmitting the RF signal and in disconnected 23 and connected 24 states when receiving the MR signal.

If this mechanism is applied to the integrated MRI-based AMF treatment system, the RF coil control part 5 can control the connected 23 and disconnected 24 states to be maintained in the AMF treatment mode, and the RF coils 14, 54, and 61 can change the magnetic field around the coils by using coils for transmission only or coils for both transmission and reception.

FIG. 3 is one example of an alternating voltage waveform generated by the RF coils 14, 54, and 61 that can be used in the MRI-based AMF treatment system. In FIG. 3, the unit of the x-axis is seconds (sec), which is a time unit, and the unit of the y-axis is millivolts (mV), which is a voltage unit. A substantially sinusoidal voltage waveform 17 can be generated on the RF coils 14, 54, and 61 within an electromagnetic wave frequency range of greater than 0 kHz to 380 kHz or less with alternating polarity, which does not have a harmful effect on the human body, to thereby induce an AMF. Because the RF frequency of the medium wave (right side of FIG. 3) is greater than the RF frequency of the long wave (left side of FIG. 3), the width of the electromagnetic wave signal having a frequency corresponding to the medium wave is shown narrower than the width of the electromagnetic wave signal having a frequency corresponding to the long wave.

The gradient coil signal transmission part 9 including an amplifier for the gradient coil transmission signal can transmit a control sequence signal 17 to the gradient coils 51, 52, and 53 to thereby drive them according to the control of the gradient coil control part 6 in order to change the magnetic field in the x-axis, y-axis, and z-axis directions around the gradient coils 51, 52, and 53.

The control sequence signal 17 transmitted to the gradient coils 51, 52, and 53 in order to change the magnetic field around the gradient coils 51, 52, and 53 can be represented by a gradient waveform. Here, the gradient waveform can be set by, basically, adjusting parameters such as the change in the magnitude of the gradient magnetic field (mT/m) over time (sec) and the duration.

Referring to FIG. 4, an embodiment of a sequence waveform using a sinusoidal gradient waveform to drive the gradient coils 51, 52, and 53 over time is shown. A substantially sinusoidal waveform 18 may be generated on a gradient magnetic field axis of each of x, y, and z in a frequency range of 1 kHz to 380 kHz with alternating polarity, to thereby induce an AMF.

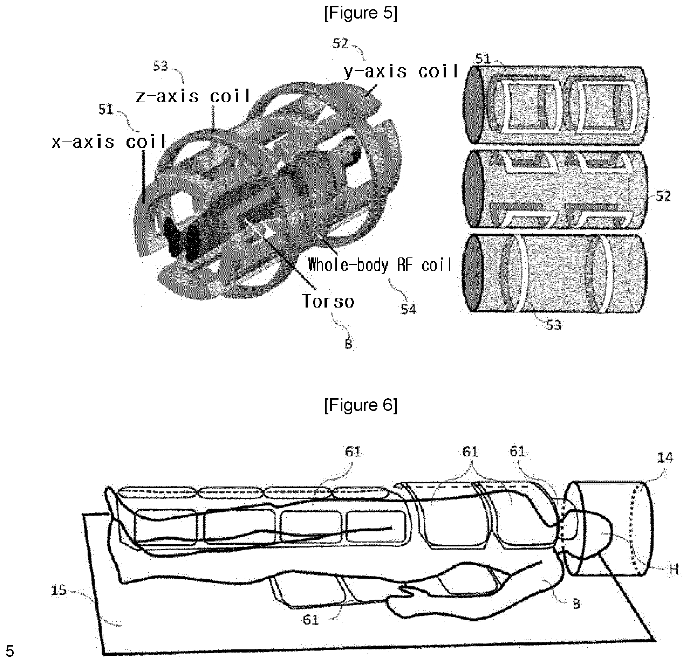

Referring to FIG. 5, the gradient coils 51, 52, and 53 may be arranged in pairs along a predetermined direction that can generate gradient magnetic fields in the x-, y-, and z-axis directions that are orthogonal to each other, to thereby change the magnetic fields in the x, y, and z axes three-dimensionally. Accordingly, an AMF can be generated by surrounding the target area of the whole body of the patient. The RF coils 14, 54, and 61 may be in a form 54 fixed to the MRI gantry 10, or may be in a form 14 and 61 that is detachable. Referring to FIG. 6, the detachable RF coils 14 and 61 may include RF coils 14 and 61 for parts of the body including the head, neck, chest, waist, knees, etc. Therefore, an AMF can be generated by surrounding local target areas of the patient through the detachable RF coils 14 and 61.

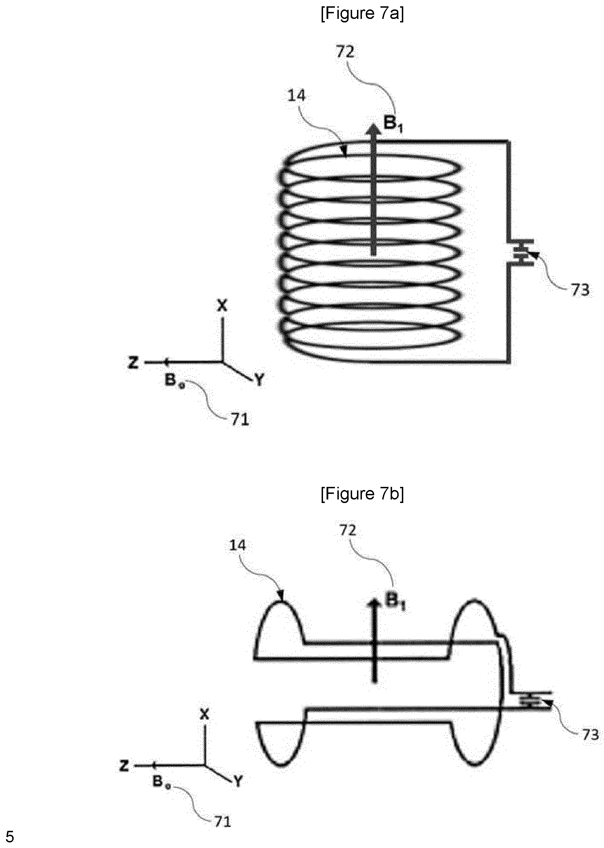

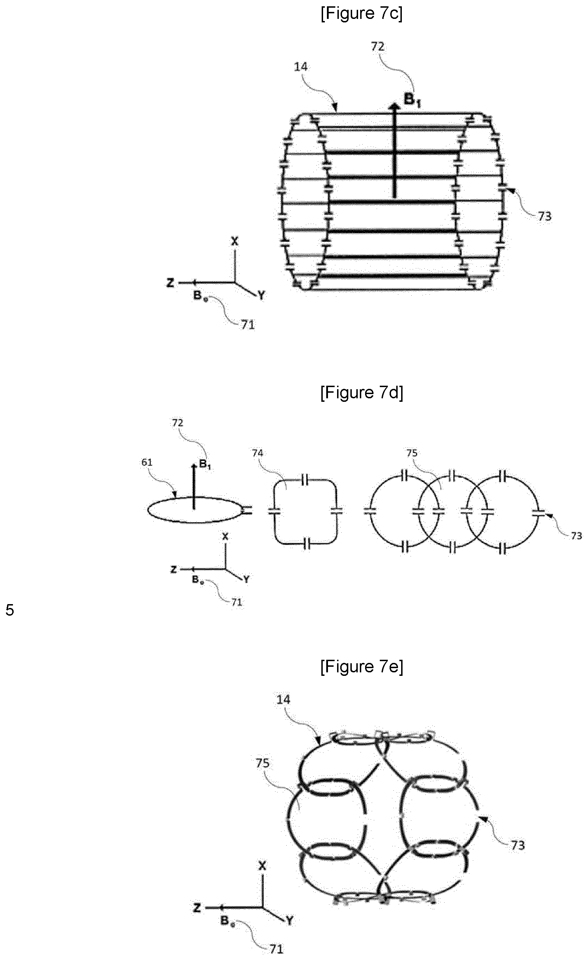

In addition, the RF coils may include not only structures such as volume coils (FIGS. 7a, 7b, and 7c), surface-attached coils (FIG. 7d), and the like, but also phased array coils (FIG. 7e) that can have various channel configurations depending on the number of channels, and thus can generate an AMF according to various treatment purposes.

Further, the MRI-based AMF treatment system according to an embodiment of the present disclosure may not only have the MRI device and the AMF generating part, i.e., the MRI scanning part and the AMF treatment part integrated structurally (integrated system), but also be configured in a fusion-type system in which each of these may be manufactured separately and AMF treatment and MRI scanning can be performed with a time difference in a shuttle fashion, for example. In the following, such a fusion-type system will be described in detail.

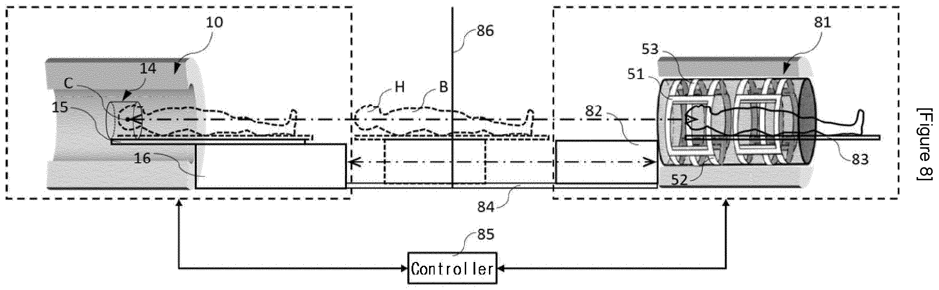

As shown in FIG. 8, a fusion-type MRI-based AMF treatment system according to one embodiment of the present disclosure is configured by including an MRI device 10, an MRI-based AMF treatment device 81, and a controller 85. Further, the AMF treatment device according to the system may include an AMF coil system for generating a predetermined alternating magnetic field, and such an AMF coil system may not only have an MRI coil system (e.g., gradient coils and/or RF coils), which is installed in a typical MRI device as described above, applied thereto, but also have any coil system, which generates an alternating magnetic field, applied thereto, without being necessarily limited.

The MRI device 10 and the MRI-based AMF treatment device 81 may be housed in respective zones that are spatially separated from each other, and each zone may be partitioned by a shielding member 86 that can be opened and closed.

According to one embodiment of the present disclosure, the MRI device 10 and the MRI-based AMF treatment device 81 may be operated independently in each zone, or the MRI device 10 and the MRI-based AMF treatment device 81 may be operated in conjunction or fusion, depending on clinical needs.

A rail part 84 may be formed at the bottom of the MRI scanning zone and the MRI-based AMF treatment zone. A transfer unit may be mounted on the bottom surface of a shuttle bed 82 that can move reciprocatingly between the two zones, and the transfer unit mounted on the bottom surface of the shuttle bed 82 may contact a guide rail formed on the upper surface of the rail part 84 and move in the longitudinal direction, or engage with a rack and move in the longitudinal direction.

As another alternative example, the upper surface of the rail part 84 may be provided with a rack extending along the longitudinal direction of the rail part 84, and the bottom surface of the shuttle bed 82 may be provided with a rack gear. Thereby, the rack gear of the shuttle bed 82 may engage with the rack provided on the upper surface of the rail part 84 and move along the longitudinal direction of the rail part 84. The transfer unit of the shuttle bed 82 may include a rack gear and a drive unit for driving the rack gear.

According to one embodiment of the present disclosure, the rail part 84 may be installed in a straight line from the rear of the MRI-based AMF treatment system 81 to the front of a front sub-bed 16 of the existing MRI located on the opposite side, without the need for the shuttle bed 82 to rotate to change the body orientation between movements.

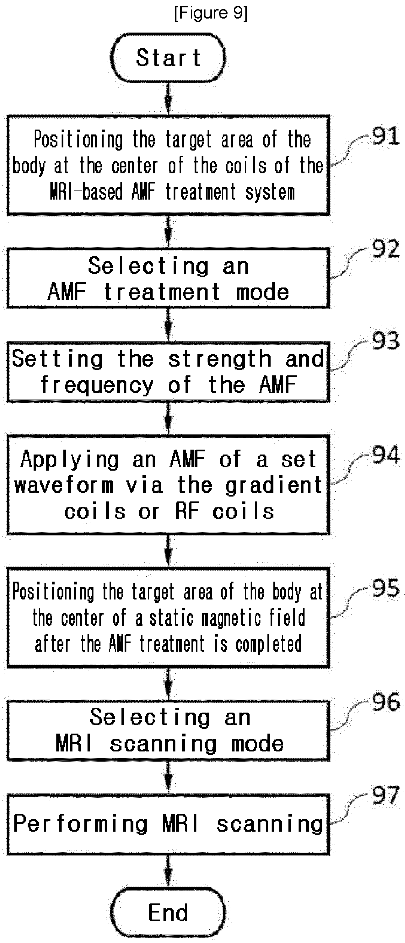

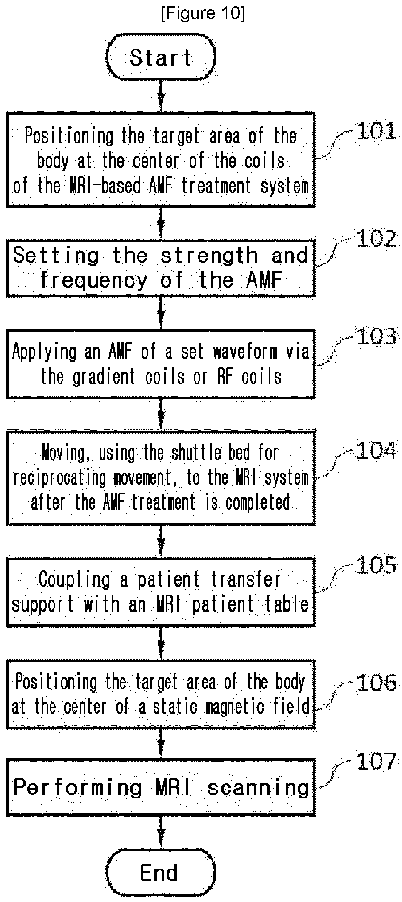

Accordingly, the shuttle bed 82 can move reciprocatingly to each zone along the rail part 84 according to the control of the controller 85. Further, the controller 85 of the shuttle bed 82 may be configured integrally with the system control part 2 described above. An AMF treatment and MRI scanning process according to one embodiment of the present disclosure is shown in FIG. 9 (integrated) and FIG. 10 (fusion type), respectively, using flowcharts. Referring to FIG. 9, the operation process in the integrated MRI-based AMF treatment system according to the present embodiment may consist of the following steps.

In step 91, the system control part 2 positions the target area of the body at the center of the gradient coils 51, 52, and 53 or the RF coils 14, 54, and 61 by controlling the position of the table 15 according to a user command input from the operating computer 1.

In step 92, the system control part 2 selects an AMF treatment mode according to a user command input from the operating computer 1 and selects the transmission/reception switch 22 to maintain the transmission direction.

In step 93, the system control part 2 sets all parameter information necessary to control the gradient coils 51, 52, and 53 or RF coils 14, 54, and 61, such as a particular frequency and strength, time information, etc., within an electromagnetic wave frequency range of 380 kHz or less with the alternating polarity of AMF waveforms 17 and 18 according to one embodiment shown in FIGS. 3 and 4, according to a user command input from the operating computer 1.

In step 94, the system control part 2 applies an electromagnetic wave signal having the AMF waveform selected in step 93 to the target area of the body using the gradient coils 51, 52, and 53 or the RF coils 14, 54, and 61 by controlling the RF coil signal transmission part 8 or the gradient coil signal transmission part 9.

In step 95, after the AMF treatment is completed, the system control part 2 positions the target area of the body at the center C of a static magnetic field by controlling the position of the table 15 according to a user command input from the operating computer 1.

In step 96, the system control part 2 selects an MRI scanning mode according to a user command input from the operating computer 1, and sets all parameter information so that the transmission/reception switch 22 operates according to an MRI pulse sequence.

In step 97, data of the MR signals received by the RF coils 14, 54, and 61 are collected. A series of MRI scanning operations for generating MR images are performed using the data of the MR signals collected.

Referring to FIG. 10, the operation process in the fusion-type MRI-based AMF treatment system according to the present embodiment may consist of the following steps.

Step 101 is the same as step 91.

Step 102 is the same as step 93.

Step 103 is the same as step 94.

In step 104, after the AMF treatment is completed, the system control part 2 moves a patient transfer support 83 on the upper surface of the table 15 of the MRI-based AMF treatment system 81 onto the upper surface of the shuttle bed 82 according to a user command input from the operating computer 1, and then the controller 85 controls the shielding member 86 that opens and closes the two zones to open. Next, the controller 85 moves the shuttle bed 82 including the patient transfer support 83 along the longitudinal direction of the rail part 84 to the end of the rail part 84 installed up to the front of the front sub-bed 16 of the existing MRI device 10 zone. Next, the controller 85 controls the shielding member 86 to close for safety.

In step 105, the controller 85 controls the patient transfer support 83 on the upper surface of the shuttle bed 82 to move onto the upper surface of the table 15 of the existing MRI device 10, and then couples them.

In step 106, the system control part 2 positions the target area of the body at the center C of the static magnetic field by controlling the position of the table 15 according to a user command input from the operating computer 1.

In step 107, the system control part 2 sets all parameter information to operate according to an MRI pulse sequence input from the operating computer 1, and then collects data of the MR signals received by the RF coils 14, 54, and 61. Next, a series of MRI scanning operations for generating MR images are performed using the data of the MR signals collected.

Further, it is obvious that the direction of the flowcharts shown in FIGS. 9 and 10 according to one embodiment of the present disclosure is not necessarily limited to the direction of the MRI scanning after the AMF treatment, and the flow in the reverse direction is also possible.

The methods in accordance with the embodiments may be implemented in the form of program commands that can be executed via a variety of computer means and may be recorded on a computer-readable medium. The computer-readable medium may include program commands, data files, data structures, and the like, alone or in combination. The program commands to be recorded on the medium may be those specially designed and configured for the embodiments, or those known and available to a person of ordinary skill in the art of computer software. Examples of the computer-readable recording medium include magnetic media such as hard disks, floppy disks, and magnetic tapes, optical media such as CD-ROMs and DVDs, magneto-optical media such as floptical disks, and hardware devices specially configured to store and execute program commands, such as ROM, RAM, flash memory, etc. Examples of the program commands include not only machine language code, such as those created by a compiler, but also high-level language code that can be executed by a computer using an interpreter or the like. The hardware devices described above may be configured to operate as one or more software modules, and vice versa, to carry out the operations of the embodiments.

As above, although the embodiments have been described by way of limited embodiments and drawings, those having ordinary skill in the art can make various modifications and variations from the above description. For example, appropriate results can be achieved even if the described techniques are performed in a different sequence from the described methods, and/or the components of the described systems, structures, devices, circuits, and the like are incorporated or combined in a form different from the described methods, or are replaced or substituted by other components or equivalents.

Therefore, other implementations, other embodiments, and equivalents of the claims also fall within the scope of the claims set forth below.

## Claims

1. A magnetic resonance imaging (MRI)-based alternating magnetic field (AMF) treatment system, comprising:
an MRI device including an MRI coil system,
wherein the MRI-based AMF treatment system is configured to generate an AMF by applying an alternating current to the MRI coil system in the MRI device.

2. A magnetic resonance imaging (MRI)-based alternating magnetic field (AMF) treatment system, comprising:
an MRI device; and
an AMF generating part operable in conjunction with the MRI device and configured to generate an AMF,
wherein the system is configured to operate between an MRI scanning mode and an AMF treatment mode.

3. The MRI-based AMF treatment system of claim 2, wherein MRI scanning and AMF treatment are configured to be performed in a single space.

4. The MRI-based AMF treatment system of any one of claims 1 to 3, wherein the MRI device comprises:
an MRI coil system configured to surround a target area of a whole body of a patient and generate an AMF;
a signal transmission/reception part configured to apply an alternating current to the MRI coil system; and
a system control part configured to switch between the MRI scanning mode and the AMF treatment mode and to adjust a strength and a frequency of the AMF.

5. The MRI-based AMF treatment system of claim 4, wherein the MRI coil system comprises at least one or more of a gradient coil and an RF coil.

6. The MRI-based AMF treatment system of claim 5, wherein the gradient coil is configured to surround the target area of the whole body of the patient and change magnetic fields in x, y, and z axes three-dimensionally, and the RF coil is configured to surround a local target area of the patient and generate an AMF.

7. The MRI-based AMF treatment system of claim 5, wherein the coil is arranged in a plurality of parallel structures.

8. The MRI-based AMF treatment system of claim 5, wherein the coil has a plurality of different inner diameters.

9. The MRI-based AMF treatment system of claim 4, wherein the MRI-based AMF treatment system is configured to operate in one of the MRI scanning mode and the AMF treatment mode, and to operate the signal transmission/reception part via an existing MRI sequence controller when operating in the MRI scanning mode, and to transmit a signal to and operate the gradient coil or the RF coil according to a sequence designed separately so that an alternating current is supplied to an MRI coil when operating in the AMF treatment mode.

10. The MRI-based AMF treatment system of claim 4, wherein the system control part is configured to flow an alternating current having a set frequency through coils, convert a surrounding magnetic field into an AMF, and thereby generate the AMF in the target area of the whole body of the patient.

11. The MRI-based AMF treatment system of claim 10, wherein the set frequency has an electromagnetic wave range of 380 kHz or less.

12. The MRI-based AMF treatment system of claim 4, wherein the system control part controls one of the MRI scanning mode and the AMF treatment mode to be closed in a switched-on or switched-off control method.

13. The MRI-based AMF treatment system of claim 4, wherein the system control part controls a table on which the patient is positioned so that an MRI scanning site is drawn into an isocenter of a static magnetic field when the MRI-based AMF treatment system completes driving the AMF treatment mode, and controls the table on which the patient is positioned to be drawn out of the MRI-based AMF treatment system when treatment and diagnosis via the MRI-based AMF treatment system are completed.

14. The MRI-based AMF treatment system of claim 2, wherein MRI scanning by the MRI device and AMF treatment by the AMF generating part are configured to be performed in spaces separated from each other.

15. The MRI-based AMF treatment system of claim 14, wherein the AMF generating part comprises an AMF coil system configured to surround a target area of a whole body of a patient and generate an AMF, and
wherein the MRI-based AMF treatment system further comprises:
a shuttle bed configured to be movable to separated spaces according to the MRI scanning mode and the AMF treatment mode;
a signal transmission/reception part configured to apply an alternating current to at least the AMF coil system; and
a system control part configured to control operation of the shuttle bed, the MRI device, and the AMF generating part and to adjust a strength and a frequency of the AMF according to each mode switching.

16. The MRI-based AMF treatment system of claim 15, wherein the AMF coil system comprises at least one or more of a gradient coil and an RF coil, the gradient coil is configured to surround the target area of the whole body of the patient and change magnetic fields in x, y, and z axes three-dimensionally, and the RF coil is configured to surround a local target area of the patient and generate an AMF.

17. The MRI-based AMF treatment system of claim 15, wherein the coil is arranged in a plurality of parallel structures, or
the coil has a plurality of different inner diameters.

18. The MRI-based AMF treatment system of claim 15, wherein the frequency of the AMF has an electromagnetic wave range that does not have a harmful effect on a human body.

19. A method of operating an MRI-based AMF treatment system, comprising:
selecting to operate in one of an MRI scanning mode and an AMF treatment mode according to an input user command; and
controlling a position of a table according to a selected mode.

20. The method of claim 19, wherein a signal transmission/reception part is operated via an existing MRI sequence controller when operating in the MRI scanning mode, and a signal is transmitted to a coil according to a sequence designed to supply an alternating current to the coil configured to generate an AMF, thereby operating the coil when operating in the AMF treatment mode.

21. The method of claim 19, comprising:
in the case of selecting the AMF treatment mode,
controlling the position of the table so that a target area of a body is located at a center of a coil for generating an AMF;
setting parameters including a strength and a frequency of an AMF waveform; and
generating an electromagnetic wave signal having a set AMF waveform through the coil.

22. The method of claim 21, further comprising:
after the generating the electromagnetic wave signal having the set AMF waveform,
controlling the position of the table so that the target area of the body is located at a center of a static magnetic field of an MRI device; and
performing an MRI scanning operation.

23. The method of any one of claims 19 to 22, wherein MRI scanning and AMF treatment are configured to be performed in a single space.

24. The method of claim 23, wherein the table is a table of an MRI device, and the coil is an MRI coil of the MRI device.

25. The method of any one of claims 19 to 22, wherein MRI scanning and AMF treatment are configured to be performed in each of spaces that are spaced and separated from each other.

26. The method of claim 25, wherein a position control of the table comprises being performed by a shuttle bed configured to be movable through separated spaces according to the MRI scanning mode and the AMF treatment mode.

27. The method of claim 19, wherein the MRI-based AMF treatment system comprises an MRI device and an AMF treatment device capable of performing the MRI scanning mode and the AMF treatment mode independently of each other, and
wherein the method further comprises:
controlling a position of a shuttle bed capable of moving reciprocatingly between the connected MRI device and the AMF treatment device, after completion of one of the MRI scanning mode and the AMF treatment mode; and
coupling a patient transfer support on the shuttle bed with a patient table.

28. A non-transitory computer-readable medium, wherein the medium comprises instructions that cause a processor to perform the method of operating according to any one of claims 19 to 22.
